(19) Europäisches Patentamt
European Patent Office
Office européen des brevets

(11) EP 0 782 988 A1

(12) **EUROPEAN PATENT APPLICATION**
published in accordance with Art. 158(3) EPC

(43) Date of publication:
**09.07.1997 Bulletin 1997/28**

(21) Application number: **96923100.0**

(22) Date of filing: **16.07.1996**

(51) Int. Cl.[6]: **C07D 209/42**, C07D 401/06, C07D 403/06, C07D 405/06, C07D 409/06, A61K 31/40, A61K 31/41, A61K 31/44, A61K 31/445, A61K 31/535

(86) International application number:
**PCT/JP96/01979**

(87) International publication number:
**WO 97/03964 (06.02.1997 Gazette 1997/07)**

(84) Designated Contracting States:
**AT BE CH DE DK ES FI FR GB GR IE IT LI LU MC NL PT SE**

(30) Priority: **18.07.1995 JP 181950/95**

(71) Applicant: **KYOWA HAKKO KOGYO CO., LTD.**
**Chiyoda-ku, Tokyo 100 (JP)**

(72) Inventors:
- **MACHII, Daisuke**
**Shizuoka 411 (JP)**
- **TAKAI, Haruki**
**Kanagawa 246 (JP)**
- **SUZUKI, Koji**
**Shizuoka 411 (JP)**
- **KOSAKA, Nobuo**
**Shizuoka 411 (JP)**
- **SATO, Soichiro**
**Shizuoka 411 (JP)**

(74) Representative: **VOSSIUS & PARTNER**
**Postfach 86 07 67**
**81634 München (DE)**

# (54) INDOLE DERIVATIVES

(57) The present invention relates to indole derivatives represented by formula (I):

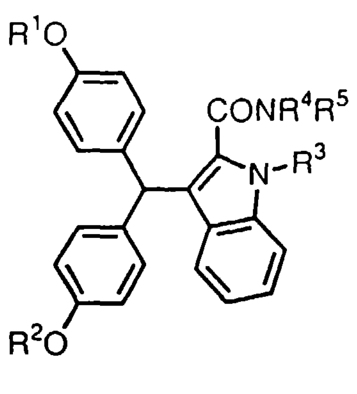

(I)

wherein $R^1$ and $R^2$ independently represent hydrogen, lower alkyl, $-O-(CH_2)_n-OR^6$, or

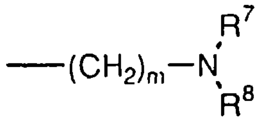

$R^3$ represents hydrogen, lower alkyl, or

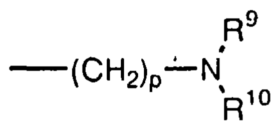

and $R^4$ and $R^5$ represent hydrogen, lower alkyl, alicyclic alkyl, substituted or unsubstituted aryl, a substituted or unsubstituted heterocyclic group,

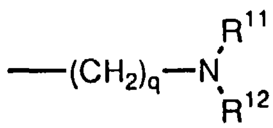

or $-(CH_2)_r-R^{13}$, or pharmaceutically acceptable salts thereof.

EP 0 782 988 A1

## Description

### Technical Field

The present invention relates to indole derivatives which are useful as therapeutic agents for osteoporosis.

### Background Art

It is disclosed in Japanese Published Unexamined Patent Application No. 215461/91 that a triphenylmethane derivative having a substituent such as carbamoyl is useful as a therapeutic agent for osteoporosis. Further, it is disclosed in Japanese Published Unexamined Patent Application No. 211651/92 that an indole derivative having a phenyl group is useful as a therapeutic agent for osteoporosis. Further, an indole derivative having a carbamoyl group is disclosed in Japanese Published Unexamined Patent Application No. 76586/95.

### Disclosure of the Invention

The present invention relates to indole derivatives represented by formula (I):

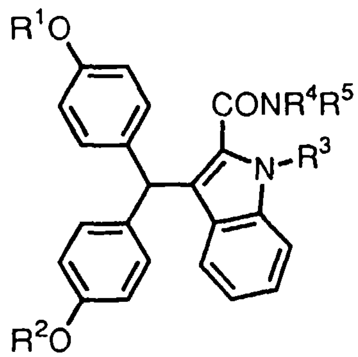

(I)

wherein $R^1$ and $R^2$ independently represent hydrogen, lower alkyl, $-O-(CH_2)_n-OR^6$ (wherein $R^6$ represents hydrogen or lower alkyl, and n represents an integer of 1 to 6), or

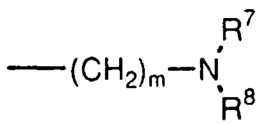

(wherein $R^7$ and $R^8$ independently represent hydrogen or lower alkyl, or $R^7$ and $R^8$ are combined together with the adjacent nitrogen atom to form a substituted or unsubstituted alicyclic heterocyclic group, and m represents an integer of 2 to 6), $R^3$ represents hydrogen, lower alkyl, or

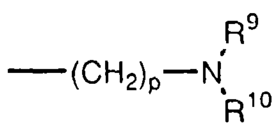

(wherein $R^9$ and $R^{10}$ independently represent hydrogen or lower alkyl, or $R^9$ and $R^{10}$ are combined together with the adjacent nitrogen atom to form a substituted or unsubstituted alicyclic heterocyclic group, and p represents an integer of 2 to 6), $R^4$ represents hydrogen, lower alkyl, alicyclic alkyl, substituted or unsubstituted aryl, a substituted or unsubstituted heterocyclic group,

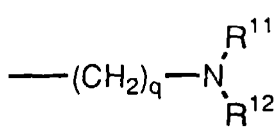

(wherein $R^{11}$ and $R^{12}$ independently represent hydrogen or lower alkyl, or $R^{11}$ and $R^{12}$ are combined together with the adjacent nitrogen atom to form a substituted or unsubstituted alicyclic heterocyclic group, and q represents an integer of 2 to 6), or $-(CH_2)_r-R^{13}$ (wherein $R^{13}$ represents substituted or unsubstituted aryl or a substituted or unsubstituted heterocyclic group, and r represents an integer of 1 to 6), and $R^5$ represents

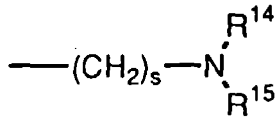

(wherein $R^{14}$ and $R^{15}$ independently represent hydrogen or lower alkyl, or $R^{14}$ and $R^{15}$ are combined together with the adjacent nitrogen atom to form a substituted or unsubstituted alicyclic heterocyclic group, and s represents an integer of 2 to 6), or $-(CH_2)_t-R^{16}$ (wherein $R^{16}$ represents substituted or unsubstituted aryl or a substituted or unsubstituted heterocyclic group, and t represents an integer of 1 to 6), or pharmaceutically acceptable salts thereof.

The compounds represented by formula (I) are hereinafter referred to as Compound (I). The same applies to the compounds of other formula numbers.

In the definitions of the groups in formula (I), the lower alkyl means a straight or branched alkyl group having 1 to 8 carbon atoms such as methyl, ethyl, propyl, isopropyl, butyl, isobutyl, sec-butyl, tert-butyl, pentyl, neopentyl, hexyl, heptyl, and octyl. The alicyclic alkyl means an alicyclic alkyl group having 3 to 8 carbon atoms such as cyclopropyl, cyclobutyl, cyclopentyl, cyclohexyl, cycloheptyl, and cyclooctyl. The aryl means phenyl and naphthyl. The alicyclic heterocyclic group means a group such as pyrrolidinyl, imidazolidinyl, pyrazolidinyl, piperidino, piperazinyl, homopiperazinyl, morpholino, and thiomorpholino. The heterocyclic group means a group such as an aromatic heterocyclic group (e.g., pyridyl, pyrazinyl, pyrimidinyl, pyridazinyl, quinolyl, isoquinolyl, phthalazinyl, naphthylidinyl, quinoxalinyl, thienyl, furyl, pyrrolyl, imidazolyl, pyrazolyl, triazolyl, tetrazolyl, thiazolyl, oxazolyl, indolyl, indazolyl, benzimidazolyl, purinyl), pyranyl, piperidyl, and tetrahydrofuranyl, in addition to the above-mentioned alicyclic heterocyclic groups.

The substituted alicyclic heterocyclic group, the substituted heterocyclic group, and the substituted aryl each has 1 to 3 independently selected substituents. Examples of the substituents are lower alkyl, hydroxy, lower alkoxy, lower alkylthio, aralkyl, carboxy, lower alkoxycarbonyl, lower alkanoyl, aroyl, halogen, nitro, amino, mono- or di(lower alkyl)amino, trifluromethyl, oxo, substituted or unsubstituted phenyl, pyridyl, and pyrimidinyl.

In the definitions of the substituents, the lower alkyl and the lower alkyl moiety of the lower alkoxy, lower alkylthio, lower alkoxycarbonyl, and mono- or di(lower alkyl)amino have the same meaning as defined for the above-mentioned lower alkyl. The aralkyl means an aralkyl group having 7 to 13 carbon atoms such as benzyl, phenethyl, benzhydryl, and naphthylmethyl. The lower alkanoyl means an alkanoyl group having 1 to 7 carbon atoms such as formyl, acetyl, propionyl, butyryl, isobutyryl, valeryl, pivaloyl, hexanoyl, and heptanoyl. The aryl moiety of the aroyl has the same meaning as defined for the above-mentioned aryl. The halogen means fluorine, chlorine, bromine, and iodine. The substituted phenyl has 1 to 3 independently selected substituents such as lower alkyl, hydroxy, lower alkoxy, lower alkylthio, aralkyl, carboxy, lower alkoxycarbonyl, lower alkanoyl, aroyl, halogen, nitro, amino, mono- or di(lower alkyl)amino, and trifluoromethyl. The lower alkyl, lower alkoxy, lower alkylthio, aralkyl, lower alkoxy carbonyl, lower alkanoyl, aroyl, halogen, and mono- or di(lower alkyl)amino have the same definitions as defined above.

The pharmaceutically acceptable salts of Compound (I) include inorganic acid addition salts such as hydrochloride, sulfate, nitrate, and phosphate, organic acid addition salts such as acetate, maleate, fumarate, tartrate, citrate, lactate, glyoxylate, aspartate, methanesulfonate, ethanesulfonate, and benzenesulfonate, metal salts such as sodium salt, potassium salt, and calcium salt, ammonium salt, tetramethylammonium salt, and amine addition salts such as a salt with morpholine.

The processes for producing Compound (I) are described below.

Compound (I) can be prepared according to the following reaction step:

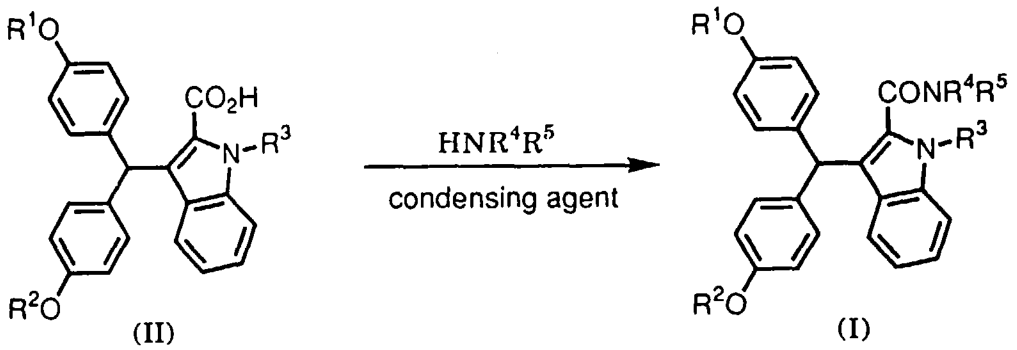


(II) (I)

(In the formulae, $R^1$, $R^2$, $R^3$, $R^4$, and $R^5$ have the same meanings as defined above.)

Compound (I) can be obtained by reacting Compound (II) with a primary amine or a secondary amine in the presence of a condensing agent such as dicyclohexylcarbodiimide and 1-(3-dimethylaminopropyl)-3-ethylcarbodiimide hydrochloride, in a solvent such as methylene chloride, chloroform, and tetrahydrofuran, at a temperature between room temperature and the boiling point of the employed solvent for 1 to 24 hours.

Compound (Ib), which is Compound (I) in which both of $R^1$ and $R^2$ are hydrogen, can also be prepared from Compound (Ia), which is Compound (I) in which both of $R^1$ and $R^2$ are methoxymethyl, according to the following reaction step:

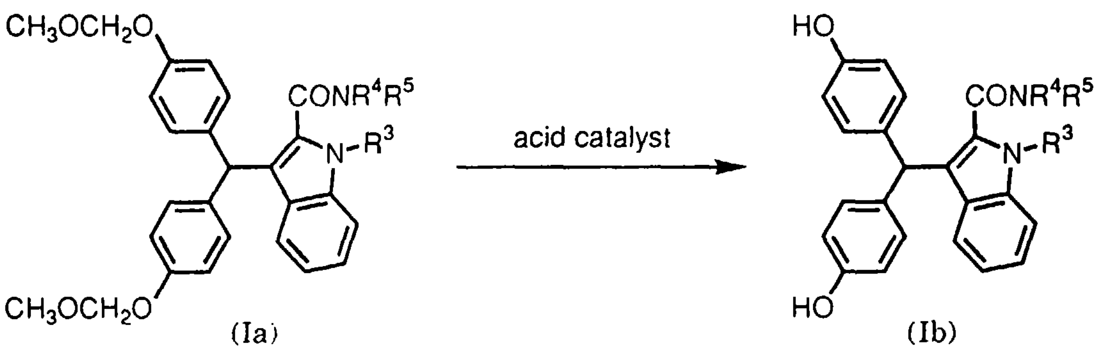


(Ia) (Ib)

(In the formulae, $R^3$, $R^4$, and $R^5$ have the same meanings as defined above.)

Compound (Ib) can be obtained by treating Compound (Ia) in the presence of an acid catalyst such as hydrochloric acid, sulfuric acid, and paratoluenesulfonic acid, in a solvent such as a lower alcohol (e.g., methanol, ethanol), N,N-dimethylformamide, dimethyl sulfoxide, tetrahydrofuran, and water, or a mixed solvent thereof, at a temperature between room temperature and the boiling point of the employed solvent for 0.5 to 24 hours.

The processes for producing starting Compounds (II) are described below.

Compound (IIa), which is Compound (II) in which all of $R^1$, $R^2$, and $R^3$ are groups other than hydrogen, can be prepared from Compound (IIIa) and Compound (1) according to the following reaction steps:

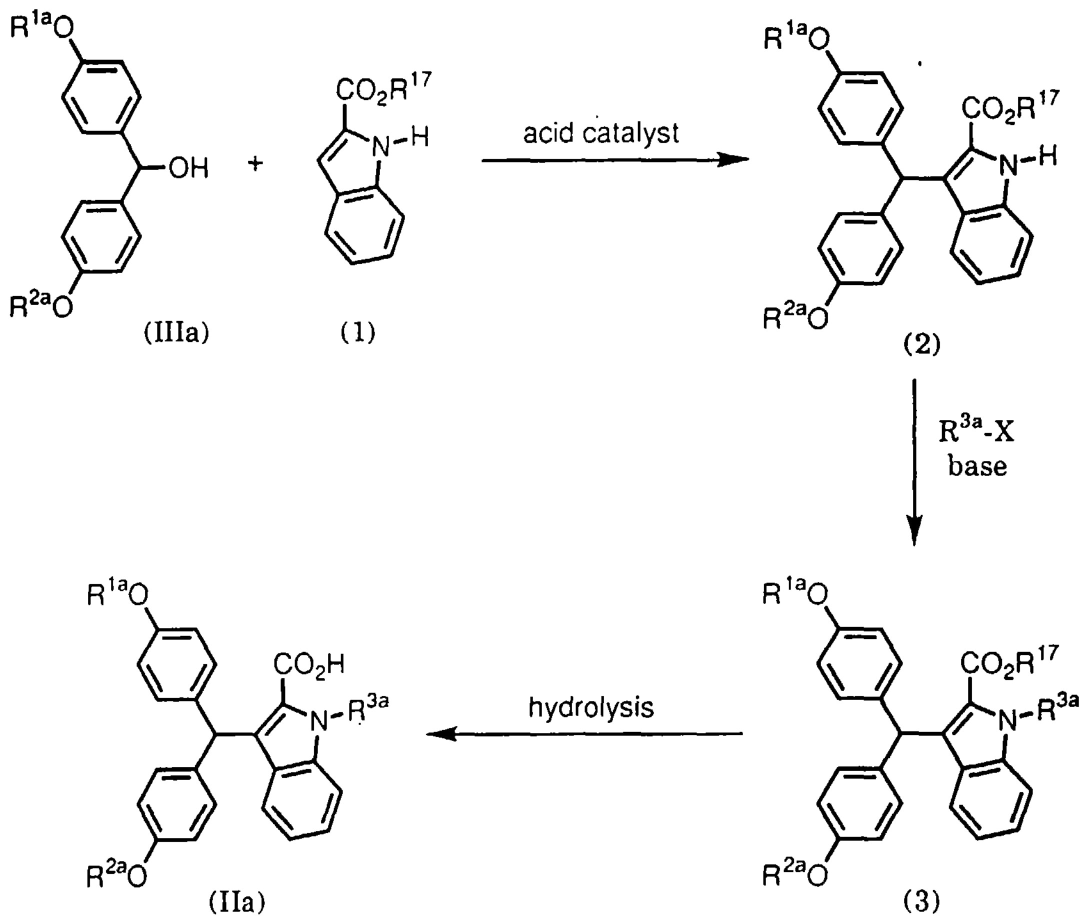


(IIIa) (1) (2) (3) (IIa)

(In the formulae, $R^{1a}$, $R^{2a}$, and $R^{3a}$ each represents a group other than hydrogen in the definition of $R^1$, $R^2$, and $R^3$, $R^{17}$ represents lower alkyl, and X represents chlorine, bromine, or iodine.)

The lower alkyl in the definition of $R^{17}$ has the same meaning as defined above.

Compound (2) can be obtained by reacting Compound (IIIa) obtained according to the method described in Tetrahedron Lett., 28, 5651 (1987) or a similar method thereto with Compound (1) obtained according to the method described in Chem. Pharm. Bull., 21, 1481 (1973) or a similar method thereto, in the presence of 0.1 to 1 equivalence of an acid catalyst such as boron trifluorideether complex, methanesulfonic acid, paratoluenesulfonic acid, and trifluoroacetic acid, in a solvent such as methylene chloride, chloroform, ether, and tetrahydrofuran, at a temperature between -20°C and the boiling point of the employed solvent for 0.5 to 24 hours. Compound (3) can be obtained by reacting Compound (2) with a halogenated alkyl or halogenated aminoalkyl in the presence of a base such as sodium hydride and potassium tert-butoxide, in a solvent such as a lower alcohol (e.g., methanol, ethanol), N,N-dimethylformamide, dimethyl sulfoxide, and tetrahydrofuran, at a temperature between 0°C and the boiling point of the employed solvent for 0.5 to 24 hours. Then, Compound (IIa) can be obtained from Compound (3) under the normal hydrolytic condition, for example, treating Compound (3) in the presence of a base such as lithium hydroxide, sodium hydroxide, and potassium hydroxide, in a solvent such as a lower alcohol (e.g., methanol, ethanol), tetrahydrofuran, N,N-dimethylformamide, and dimethyl sulfoxide, if necessary, in the presence of water, at a temperature between room temperature and the boiling point of the employed solvent for 0.5 to 24 hours.

Compound (IIb), which is Compound (II) in which $R^1$ and $R^2$ are groups other than hydrogen and $R^3$ is hydrogen, can be prepared from Compound (2) by hydrolysis of the ester moiety according to the method of producing Compound (IIa) from Compound (3) or a similar method thereto.

Compound (IIc), which is Compound (II) in which all of $R^1$, $R^2$, and $R^3$ are hydrogen, or Compound (IId), which is Compound (II) in which $R^1$ and $R^2$ are hydrogen and $R^3$ is a group other than hydrogen, can be prepared from Compound (2a), which is Compound (2) in which both of $R^{1a}$ and $R^{2a}$ are methoxymethyl, or from Compound (3a), which is Compound (3) in which both of $R^{1a}$ and $R^{2a}$ are methoxymethyl, respectively, by carrying out demethoxymethylation according to the method of producing Compound (Ib) from Compound (Ia) or a similar method thereto, followed by hydrolysis of the ester moiety according to the method of producing Compound (IIa) from Compound (3) or a similar method thereto.

The intermediates and the desired compounds in the processes described above can be isolated and purified by purification methods conventionally used in organic synthetic chemistry, for example, filtration, extraction, washing, drying, concentration, recrystallization, and various kinds of chromatography. The intermediates may also be subjected to the subsequent reaction without purification.

In the case where a salt of Compound (I) is desired and it is produced in the form of the desired salt, it can be subjected to purification as such. In the case where Compound (I) is produced in the free state and its salt is desired, Compound (I) is dissolved or suspended in a suitable solvent, followed by addition of an appropriate acid or base to form a salt, and then the salt can be isolated.

Compounds (I) and pharmaceutically acceptable salts thereof may be in the form of adducts with water or various solvents, which are also within the scope of the present invention.

Examples of Compounds (I) obtained in the above processes are shown in Table 1.

Table 1-1

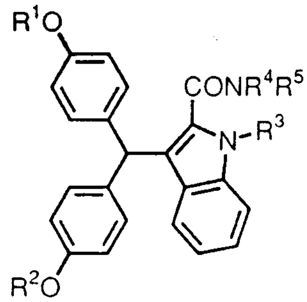

| Compd. No. | $R^1$ | $R^2$ | $R^3$ | $-NR^4R^5$ |
|---|---|---|---|---|
| 1 | $CH_2OCH_3$ | $CH_2OCH_3$ | 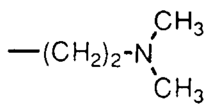 | 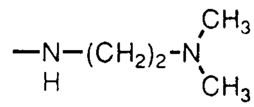 |
| 2 | $CH_2OCH_3$ | $CH_2OCH_3$ | 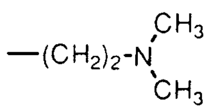 | 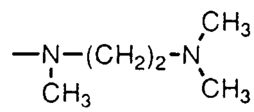 |
| 3 | $CH_2OCH_3$ | $CH_2OCH_3$ | 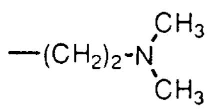 | 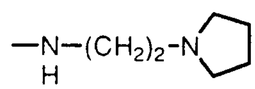 |
| 4 | $CH_2OCH_3$ | $CH_2OCH_3$ | 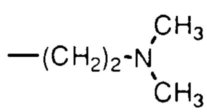 | 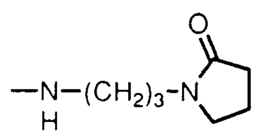 |
| 5 | $CH_2OCH_3$ | $CH_2OCH_3$ | 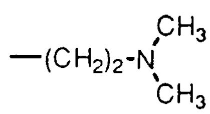 | 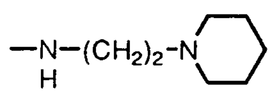 |
| 6 | $CH_2OCH_3$ | $CH_2OCH_3$ | 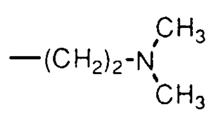 | 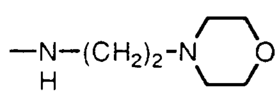 |
| 7 | $CH_2OCH_3$ | $CH_2OCH_3$ | 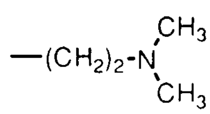 | 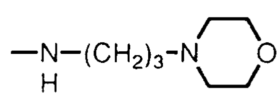 |

Table 1-2

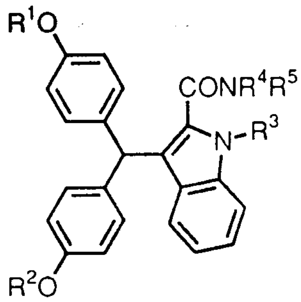

| Compd. No. | $R^1$ | $R^2$ | $R^3$ | $-NR^4R^5$ |
|---|---|---|---|---|
| 8 | $CH_2OCH_3$ | $CH_2OCH_3$ | 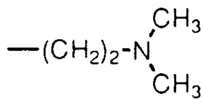 | 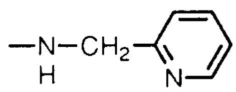 |
| 9 | $CH_2OCH_3$ | $CH_2OCH_3$ | 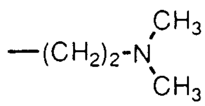 | 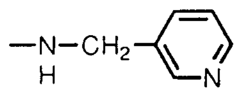 |
| 10 | $CH_2OCH_3$ | $CH_2OCH_3$ | 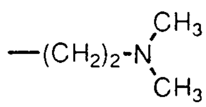 | 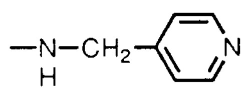 |
| 11 | $CH_2OCH_3$ | $CH_2OCH_3$ | 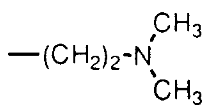 | 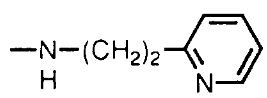 |
| 12 | $CH_2OCH_3$ | $CH_2OCH_3$ | 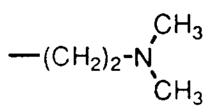 | 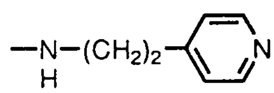 |
| 13 | $CH_2OCH_3$ | $CH_2OCH_3$ | 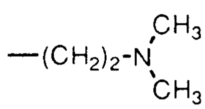 | 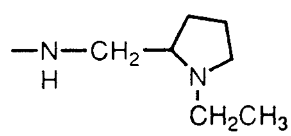 |
| 14 | $CH_2OCH_3$ | $CH_2OCH_3$ | 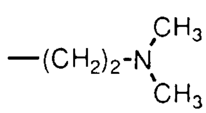 | 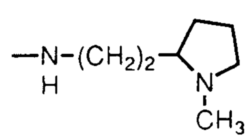 |

Table 1-3

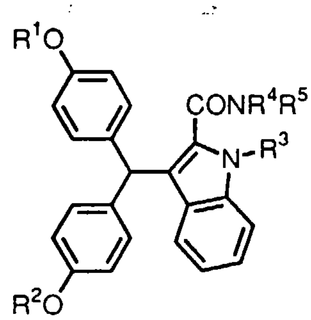

| Compd. No. | $R^1$ | $R^2$ | $R^3$ | $-NR^4R^5$ |
|---|---|---|---|---|
| 15 | $CH_2OCH_3$ | $CH_2OCH_3$ | 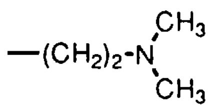 | 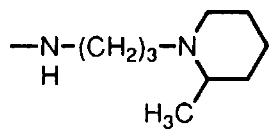 |
| 16 | $CH_2OCH_3$ | $CH_2OCH_3$ | 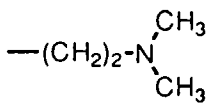 | 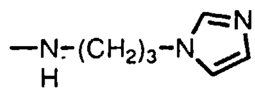 |
| 17 | $CH_2OCH_3$ | $CH_2OCH_3$ | 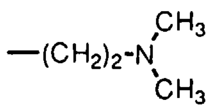 | 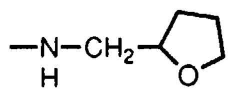 |
| 18 | $CH_2OCH_3$ | $CH_2OCH_3$ | 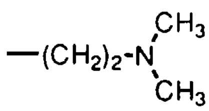 | 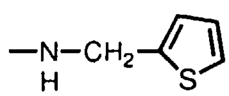 |
| 19 | $CH_2OCH_3$ | $CH_2OCH_3$ | 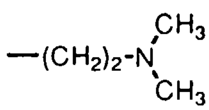 | 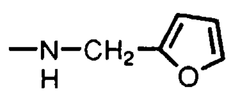 |
| 20 | $CH_2OCH_3$ | $CH_2OCH_3$ | 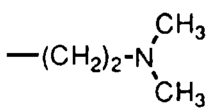 | 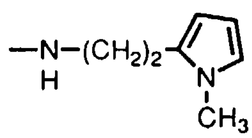 |

Table 1-4

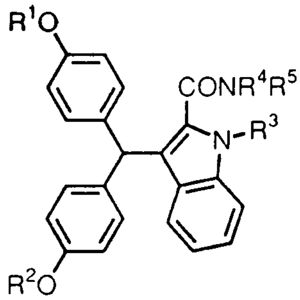

| Compd. No. | $R^1$ | $R^2$ | $R^3$ | $-NR^4R^5$ |
|---|---|---|---|---|
| 21 | H | H | 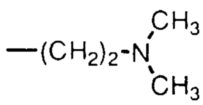 | 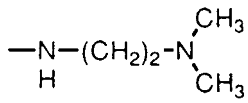 |
| 22 | H | H | 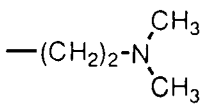 | 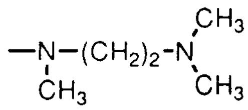 |
| 23 | H | H | 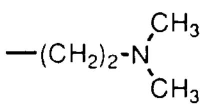 | 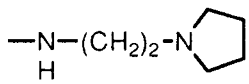 |
| 24 | H | H | 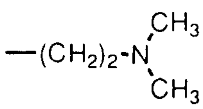 | 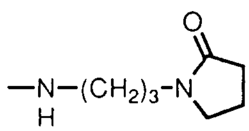 |
| 25 | H | H | 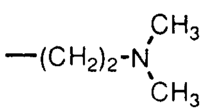 | 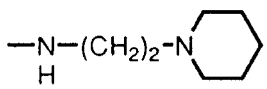 |
| 26 | H | H | 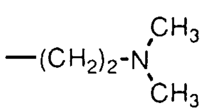 | 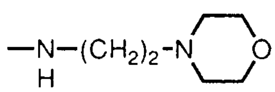 |
| 27 | H | H | 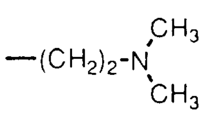 | 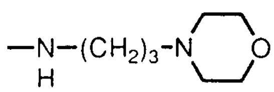 |

Table 1-5

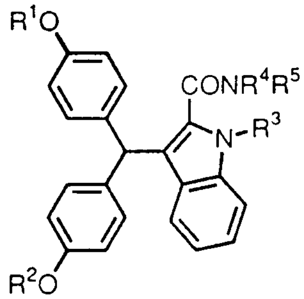

| Compd. No. | $R^1$ | $R^2$ | $R^3$ | $-NR^4R^5$ |
|---|---|---|---|---|
| 28 | H | H | 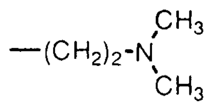 | 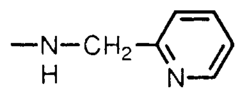 |
| 29 | H | H | 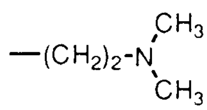 | 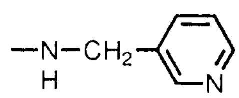 |
| 30 | H | H | 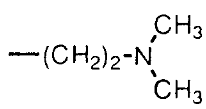 | 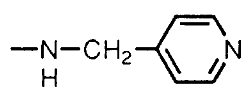 |
| 31 | H | H | 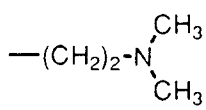 | 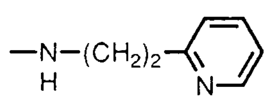 |
| 32 | H | H | 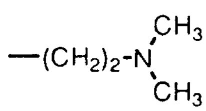 | 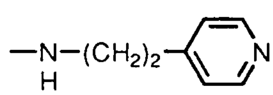 |
| 33 | H | H | 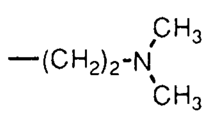 | 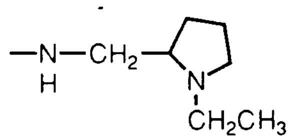 |
| 34 | H | H | 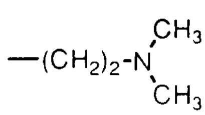 | 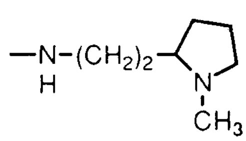 |

Table 1-6

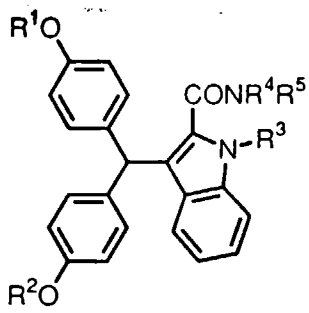

| Compd. No. | $R^1$ | $R^2$ | $R^3$ | $-NR^4R^5$ |
|---|---|---|---|---|
| 35 | H | H | 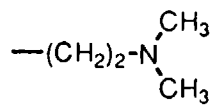 | 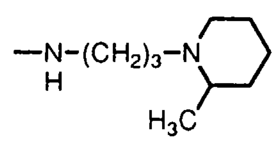 |
| 36 | H | H | 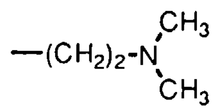 | 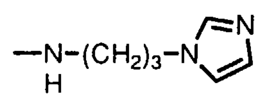 |
| 37 | H | H | 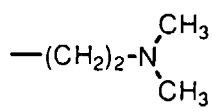 | 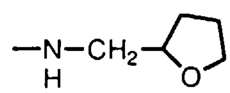 |
| 38 | H | H | 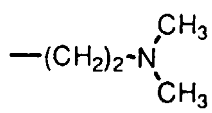 | 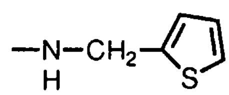 |
| 39 | H | H | 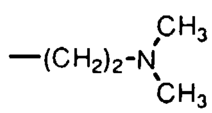 | 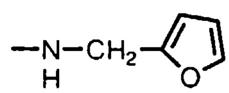 |

The inhibitory effect of the compounds of the present invention on bone absorption is shown below by test examples.

The inhibitory effect of the compounds of the present application on bone resorption was determined by their inhibitory effect on the excretion of urinary hydroxyproline which is elevated by ovariectomy of the animals tested, according to the report of Kalu et al. [Bone and Mineral., 14, 175 (1991)].

Test Example 1: Inhibitory effect on the increase of urinary hydroxyproline excretion by ovariectomy

12-weeks-old female SD strain rats (Japan Clea Co., Ltd.) were acclimated for 1 week during which they were given tap water and a standard diet (F2; Funabashi Farmas) ad libitum. The experiment was carried out using the rats weighing 270-310 g. After the animals were subjected to sham operation or bilateral ovariectomy, they were allowed free access to water passed through ion exchange resin instead of tap water, and were housed in individual cages. The test compound (10 mg/kg) was suspended in 0.3% Tween, and the obtained suspension was orally administered to the ovariectomized rats once a day for two weeks starting from the day following ovariectomy (0.5 ml/100 g body weight). 0.3% Tween was similarly administered to sham-operated rats and ovariectomized control rats. After the final administration, the rats were placed in individual metabolic cages and fasted during 24 hours, and urine was collected. The volume of the collected urine was measured and the urine was centrifuged at 3,000 rpm for 15 min at 4°C. The concentration of hydroxyproline in the supernatant was measured by the method of Ikeda et al. [Ann. Rep. Tokyo Metr. Res. Lab. P. H., 36, 277-282 (1985)] and the concentration of creatinine in the supernatant was measured with creatinine test WAKO (Wako Pure Chem.) The amount of urinary hydroxyproline excretion was expressed by the molar ratio of the amount of hydroxyproline to the amount of creatinine. The rate of inhibition on increase of the amount of urinary hydroxyproline excretion in ovariectomized rats treated with the test compound compared to that of the control group was calculated using the following equation.

$$\text{Inhibition rate (\%)} = [(P1 - P2)/(P1 - P3)] \times 100$$

P1 : The amount of urinary hydroxyproline excretion in ovariectomized rats treated with 0.3% Tween (μmol/mmol).
P2 : The amount of urinary hydroxyproline excretion in ovariectomized rats treated with the test compound (μmol/mmol).
P3 : The amount of urinary hydroxyproline in sham-operated rats treated with 0.3% Tween (μmol/mmol).

Table 2

| Compd. No. | Inhibition rate of increase of the amount of urinary hydroxyproline excretion |
|---|---|
| 22 | 100 |
| 25 | 100 |
| 27 | 30 |
| 29 | 30 |
| 30 | 100 |
| 32 | 100 |
| 33 | 100 |
| 34 | 100 |
| 37 | 100 |

Compounds (I) and pharmaceutically acceptable salts thereof can be formulated into generally employed dose forms such as tablets, capsules, and syrups, and administered orally or parenterally through intramuscular injection, intravenous injection, drip infusion, or rectal administration using suppositories. For preparing these dose forms for oral or parenteral administration, generally known techniques are applied. For example, the preparations may contain various excipients, lubricants, binders, disintegrating agents, isotonizing agents, emulsifiers, and the like.

Examples of the carriers which can be used are water, injectable distilled water, physiological saline, glucose, fructose, sucrose, mannitol, lactose, starch, cellulose, methyl cellulose, carboxymethyl cellulose, hydroxypropyl cellulose, alginic acid, talc, sodium citrate, calcium carbonate, calcium hydrogenphosphate, magnesium stearate, urea, silicone resins, sorbitan fatty acid esters, and glycerin fatty acid esters.

The effective dose and administration schedule of Compound (I) or a pharmaceutically acceptable salt thereof varies depending upon the mode of administration, the age, body weight and conditions of a patient, etc. However, generally, Compound (I) or a pharmaceutically acceptable salt thereof is administered in a dose of 0.1 to 10 mg/kg/day in 1 to 4 parts.

Certain embodiments of the present invention are illustrated in the following Examples and Reference Examples.

## Best Mode for Carrying out the Invention

### Example 1

N-(2-Dimethylaminoethyl)-3-{bis[4-(methoxymethoxy)phenyl]methyl}-1-(2-dimethylaminoethyl)indole-2-carboxamide (Compound 1)

1-(3-Dimethylaminopropyl)-3-ethylcarbodiimide hydrochloride (1.48 g, 7.71 mmol) was added to a solution of 3-(bis[4-{methoxymethoxy)phenyl]methyl}-1-(2-dimethylaminoethyl)indole-2-carboxylic acid (2.0 g, 3.86 mmol) obtained in Reference Example 3 and N,N-dimethylethylenediamine (0.64 ml, 5.78 mmol) in 30 ml of methylene chloride, followed by stirring at room temperature for 4.5 hours. Water was added to the reaction solution followed by extraction with chloroform. The resulting organic layer was washed successively with 2N hydrochloric acid, a saturated aqueous solution of sodium bicarbonate, and a saturated aqueous solution of sodium chloride, and dried over magnesium sulfate, and the solvent was distilled off under reduced pressure to give 2.48 g (quantitative) of the title compound.

$^1$H-NMR($CDCl_3$) δ(ppm): 2.14 (6H, s), 2.23 (6H, s), 2.38 (2H, t, J=6.1Hz), 2.75 (2H, t, J=6.9Hz), 3.4-3.5 (2H, m), 3.46 (6H, s), 4.43 (2H, t, J=6.9Hz), 5.14 (4H, s), 6.60 (1H, s), 6.85-6.95 (1H, m), 6.92 (4H, d, J=8.9Hz),7.01 (1H, d, J=8.3Hz), 7.12 (4H, d, J=8.9Hz), 7.15-7.25 (2H, m), 7.33 (1H,d, J=8.3Hz).

In the following Examples 2 to 20, substantially the same procedure as in Example 1 was repeated using corresponding amines in place of N,N-dimethylethylenediamine to give the desired compounds.

### Example 2

N-(2-Dimethylaminoethyl)-N-methyl-3-{bis[4-(methoxymethoxy)phenyl]methyl}-1-(2-dimethylaminoethyl)indole-2-carboxamide (Compound 2)

$^1$H-NMR($CDCl_3$) δ(ppm): 2.27 (12H, s), 2.47 (2H, t, J=6.5Hz), 2.56 (3H, s), 2.55-2.75 (2H, m), 3.44 (3H, s), 3.48 (3H, s), 3.45-3.55 (2H, m), 4.05-4.35 (2H, m), 5.11 (2H, s), 5.15 (2H, s), 5.65 (1H, s), 6.8-7.0 (5H, m), 7.08 (2H, d, J=8.6Hz), 7.15-7.3 (4H, m), 7.33 (1H, d, J=8.6Hz).

### Example 3

N-[2-(1-Pyrrolidinyl)ethyl]-3-{bis[4-(methoxymethoxy)phenyl]methyl}-1-(2-dimethylaminoethyl)indole-2-carboxamide (Compound 3)

$^1$H-NMR($CDCl_3$) δ(ppm): 1.6-1.7 (4H, m), 2.22 (6H, s), 2.4-2.5 (4H, m), 2.60 (2H, t, J=6.3Hz), 2.74 (2H, t, J=6.8Hz), 3.46 (6H, s), 3.49 (2H, q, J=6.3Hz), 4.43 (2H, t, J=6.8Hz), 5.13 (4H, s), 6.02 (1H, s), 6.85-6.95 (1H, m), 6.91 (4H, d, J=8.7Hz), 7.02 (1H, d, J=7.9Hz), 7.12 (4H, d, J=8.7Hz), 7.19 (1H, t, J=8.2Hz), 7.3-7.4 (1H, m), 7.32 (1H, d, J=8.2Hz).

### Example 4

N-[3-(2-Oxo-1-pyrrolidinyl)propyl]-3-{bis[4-(methoxymethoxy)phenyl]methyl}-1-(2-dimethylaminoethyl)indole-2-carboxamide (Compound 4)

$^1$H-NMR($CDCl_3$) δ(ppm): 1.7-1.8 (2H, m), 1.95-2.05 (2H, m), 2.19 (6H, s), 2.37 (2H, t, J=8.1Hz), 2.79 (2H, t, J=6.4Hz), 3.2-3.4 (6H, m), 3.46 (6H, s), 4.40 (2H, t, J=6.4Hz), 5.14 (4H, s), 6.07 (1H, s), 6.85-6.95 (1H, m), 6.91 (4H, d, J=8.5Hz), 7.01 (1H, d, J=7.9Hz), 7.12 (4H, d, J=8.5Hz), 7.15-7.25 (1H, m), 7.31 (1H, d, J=8.6Hz), 7.95-8.0 (1H, m).

### Example 5

N-(2-Piperidinoethyl)-3-{bis[4-(methoxymethoxy)phenyl]methyl}-1-(2-dimethylaminoethyl)indole-2-carboxamide (Compound 5)

$^1$H-NMR($CDCl_3$) δ(ppm): 1.3-1.5 (6H, m), 2.21 (6H, s), 2.3-2.4 (4H, m), 2.43 (2H, t, J=6.3Hz), 2.74 (2H, t, J=6.7Hz), 3.46 (6H, s), 3.4-3.5 (2H, m), 4.44 (2H, t, J=6.7Hz), 5.14 (4H, s), 6.02 (1H, s), 6.85-6.95 (1H, m), 6.91 (4H, d, J=8.7Hz), 7.03 (1H, d, J=7.9Hz), 7.12 (4H, d, J=8.6Hz), 7.15-7.25 (1H, m), 7.33 (1H, d, J=8.6Hz).

Example 6

N-(2-Morpholinoethyl)-3-{bis[4-(methoxymethoxy)phenyl]methyl}-1-(2-dimethylaminoethyl)indole-2-carboxamide (Compound 6)

$^1$H-NMR($CDCl_3$) δ(ppm): 2.22 (6H, s), 2.3-2.35 (4H, m), 2.46 (2H, t, J=6.1Hz), 2.75 (2H, t, J=6.6Hz), 3.45-3.6 (6H, m), 3.46 (6H, s), 4.45 (2H, t, J=6.6Hz), 5.13 (4H, s), 6.03 (1H, s), 6.85-6.95 (1H, m), 6.91 (4H, d, J=8.7Hz), 7.02 (1H, d, J=7.9Hz), 7.11 (4H, d, J=8.7Hz), 7.15-7.25 (2H, m), 7.33 (1H, d, J=8.2Hz).

Example 7

N-(3-Morpholinopropyl)-3-{bis[4-(methoxymethoxy)phenyl]methyl}-1-(2-dimethylaminoethyl)indole-2-carboxamide (Compound 7)

$^1$H-NMR($CDCl_3$) δ(ppm): 1.6-1.75 (2H, m), 2.2-2.3 (2H, m), 2.26 (6H, s), 2.3-2.4 (4H, m), 2.80 (2H, t, J=6.6Hz), 3.4-3.5 (2H, m), 3.46 (6H, s), 3.55-3.65 (4H, m), 4.43 (2H, t, J=6.6Hz), 5.14 (4H, s), 5.99 (1H, s), 6.85-7.0 (2H, m), 6.91 (4H, d, J=8.6Hz), 7.11 (4H, d, J=8.6Hz), 7.15-7.25 (1H, m), 7.3-7.4 (2H, m).

Example 8

N-(2-Pyridylmethyl)-3-{bis[4-(methoxymethoxy)phenyl]methyl}-1-(2-dimethylaminoethyl)indole-2-carboxamide (Compound 8)

$^1$H-NMR($CDCl_3$) δ(ppm): 2.18 (6H, s), 2.83 (2H, br t, J=6.1Hz), 3.46 (6H, s), 4.48 (2H, t, J=6.6Hz), 4.69 (2H, d, J=5.3Hz), 5.13 (4H, s), 6.14 (1H, s), 6.85-6.95 (1H, m), 6.88 (4H, d, J=8.7Hz), 7.0-7.1 (1H, m), 7.09 (4H, d, J=8.7Hz), 7.15-7.3 (3H, m), 7.35-7.4 (1H, m), 7.62 (1H, td, J=7.6, 1.8Hz), 8.35-8.5 (2H, m).

Example 9

N-(3-Pyridylmethyl)-3-{bis[4-(methoxymethoxy)phenyl]methyl}-1-(2-dimethylaminoethyl)indole-2-carboxamide (Compound 9)

$^1$H-NMR($CDCl_3$) δ(ppm): 2.03 (6H, s), 2.78 (2H, t, J=5.9Hz), 3.46 (6H, s), 4.40 (2H, t, J=5.9Hz), 4.56 (2H, d, J=5.6Hz), 5.13 (4H, s), 6.11 (1H, s), 6.85-7.05 (2H, m), 6.89 (4H, d, J=8.5Hz), 7.06 (4H, d, J=8.5Hz), 7.15-7.35 (3H, m), 7.5-7.6 (1H, m), 8.52 (2H, br s), 8.5-8.65 (1H, br).

Example 10

N-(4-Pyridylmethyl)-3-{bis[4-(methoxymethoxy)phenyl]methyl}-1-(2-dimethylaminoethyl)indole-2-carboxamide (Compound 10)

$^1$H-NMR($CDCl_3$) δ(ppm): 2.03 (6H, s), 2.80 (2H, t, J=5.9Hz), 3.46 (6H, s), 4.40 (2H, t, J=5.9Hz), 4.55 (2H, d, J=5.6Hz), 5.13 (4H, s), 6.20 (1H, s), 6.85-6.95 (1H, m), 6.90 (4H, d, J=8.6Hz), 7.00 (1H, d, J=7.9Hz), 7.05-7.15 (2H, m), 7.09 (4H, d, J=8.6Hz), 7.15-7.25 (2H, m), 8.51 (2H, d, J=5.9Hz), 8.85-8.95 (1H, m).

Example 11

N-[2-(2-Pyridyl)ethyl]-3-{bis[4-(methoxymethoxy)phenyl]methyl}-1-(2-dimethylaminoethyl)indole-2-carboxamide (Compound 11)

$^1$H-NMR($CDCl_3$) δ(ppm): 2.1-2.2 (6H, br), 2.75-2.85 (2H, m), 3.03 (2H, t, J=6.3Hz), 3.46 (6H, s), 3.80 (2H, q, J=6.2Hz), 4.35-4.45 (2H, m), 5.14 (4H, s), 6.02 (1H, s), 6.85-7.1 (4H, m), 6.90 (4H, d, J=8.5Hz), 7.05 (4H, d, J=8.5Hz), 7.15-7.25 (1H, m), 7.3-7.4 (1H, m), 7.46 (1H, dd, J=7.6, 2.0Hz), 7.9-8.05 (1H, br), 8.36 (1H, d, J=4.0Hz).

Example 12

N-[2-(4-Pyridyl)ethyl]-3-{bis[4-(methoxymethoxy)phenyl]methyl}-1-(2-dimethylaminoethyl)indole-2-carboxamide (Compound 12)

$^1$H-NMR($CDCl_3$) δ(ppm): 1.90 (6H, s), 2.70 (2H, t, J=5.9Hz), 2.83 (2H, t, J=6.6Hz), 3.45 (6H, s), 3.63 (2H, t, J=6.6Hz), 4.30 (2H, t, J=5.9Hz), 5.14 (4H, s), 6.20 (1H, s), 6.85-6.95 (3H, m), 6.93 (4H, d, J=8.6Hz), 7.00 (1H, d, J=8.3Hz), 7.10 (4H, d, J=8.6Hz), 7.15-7.3 (2H, m), 8.38 (2H, d, J=5.9Hz), 8.45-8.5 (1H, m).

Example 13

N-[(1-Ethyl-2-pyrrolidinyl)methyl]-3-{bis[4-(methoxymethoxy)phenyl]methyl}-1-(2-dimethylaminoethyl)indole-2-carboxamide (Compound 13)

$^1$H-NMR($CDCl_3$) δ(ppm): 0.97 (3H, t, J=7.1Hz), 1.4-1.8 (4H, m), 2.05-2.25 (2H, m), 2.19 (6H, s), 2.5-2.6 (1H, m), 2.7-2.8 (3H, m), 2.95-3.05 (1H, m), 3.15-3.25 (1H, m), 3.46 (6H, s), 3.7-3.8 (1H, m), 4.43 (2H, t, J=6.9Hz), 5.13 (4H, s), 6.01 (1H, s), 6.85-6.95 (1H, m), 6.91 (4H, d, J=8.6Hz), 7.01 (1H,d, J=7.9Hz), 7.05-7.15 (4H, m), 7.15-7.25 (1H, m), 7.33 (1H, d, J=8.3Hz).

Example 14

N-[2-(1-Methyl-2-pyrrolidinyl)ethyl]-3-{bis[4-(methoxymethoxy)phenyl]methyl}-1-(2-dimethylaminoethyl)indole-2-carboxamide (Compound 14)

$^1$H-NMR($CDCl_3$) δ(ppm): 1.4-1.55 (2H, m), 1.55-1.75 (2H, m), 1.75-1.95 (2H, m), 1.95-2.15 (2H, m), 2.22 (9H, s), 2.76 (2H, t, J=6.5Hz), 2.9-3.0 (1H, m), 3.3-3.55 (2H, m), 3.46 (6H, s), 4.41 (2H, t, J=6.5Hz), 5.14 (4H, s), 6.03 (1H, s), 6.85-6.95 (1H, m), 6.92 (4H, d, J=8.4Hz), 7.00 (1H, d, J=7.9Hz),7.11 (4H, d, J=8.4Hz), 7.15-7.25 (1H, m), 7.32 (1H, d, J=8.6Hz), 7.55-7.65 (1H, m).

Example 15

N-[3-(2-Methylpiperidino)propyl]-3-{bis[4-(methoxymethoxy)phenyl]methyl}-1-(2-dimethylaminoethyl)indole-2-carboxamide (Compound 15)

$^1$H-NMR($CDCl_3$) δ(ppm): 0.99 (3H, d, J=6.2Hz), 1.2-1.3 (2H, m), 1.3-1.8 (6H, m), 2.0-2.15 (1H, m), 2.22 (6H, s), 2.2-2.4 (2H, m), 2.65-2.8 (2H, m), 2.77 (2H, t, J=6.6Hz), 3.3-3.45 (2H, m), 3.46 (6H, s), 4.40 (2H, t, J=6.6Hz),5.14 (4H, s), 6.03 (1H, s), 6.85-6.95 (1H, m), 6.91 (4H, d, J=8.2Hz), 7.02 (1H, d, J=8.3Hz), 7.2-7.25 (4H, m), 7.25-7.35 (1H, m), 7.31 (1H, d, J=8.3Hz).

Example 16

N-[3-(1-Imidazolyl)propyl]-3-{bis[4-(methoxymethoxy)phenyl]methyl}-1-(2-dimethylaminoethyl)indole-2-carboxamide (Compound 16)

$^1$H-NMR($CDCl_3$) δ(ppm): 1.9-2.0 (2H, m), 2.24 (6H, s), 2.87 (2H, t, J=6.4Hz), 3.34 (2H, q, J=6.9Hz), 3.45 (6H, s), 3.85 (2H, t, J=6.9Hz), 4.42 (2H, t, J=6.4Hz), 5.13 (4H, s), 6.07 (1H, s), 6.85-6.95 (3H, m), 6.92 (4H, d, J=8.6Hz), 7.05-7.15 (1H, m), 7.10 (4H, d, J=8.6Hz), 7.2-7.25 (1H, m), 7.35-7.45 (2H, m), 7.65-7.8 (1H, br).

Example 17

N-(2-Tetrahydrofuranylmethyl)-3-{bis[4-(methoxymethoxy)phenyl]methyl}-1-(2-dimethylaminoethyl)indole-2-carboxamide (Compound 17)

$^1$H-NMR($CDCl_3$) δ(ppm): 1.4-2.0 (4H, m), 2.20 (6H, s), 2.7-2.85 (2H, m), 3.3-3.45 (1H, m), 3.46 (6H, s), 3.55-3.75 (3H, m), 3.9-4.0 (1H, m), 4.35-4.5 (2H, m), 5.14 (4H, s), 6.09 (1H, s), 6.85-6.95 (5H, m), 7.01 (1H, d, J=7.9Hz), 7.1-7.25 (5H, m), 7.32 (1H, d, J=8.6Hz), 7.95-8.05 (1H, m).

Example 18

N-(2-Thienylmethyl)-3-{bis-[4-(methoxymethoxy)phenyl]methyl}-1-(2-dimethylaminoethyl)indole-2-carboxamide (Compound 18)

$^1$H-NMR($CDCl_3$) δ(ppm): 2.04 (6H, s), 2.75 (2H, t, J=6.2Hz), 3.46 (6H, s), 4.41 (2H, t, J=6.2Hz), 4.74 (2H, d, J=5.3Hz), 5.13 (4H, s), 6.10 (1H, s), 6.85-7.0 (3H, m), 6.88 (4H, d, J=8.6Hz), 7.0-7.1 (1H, m), 7.07 (4H, d, J=8.6Hz), 7.15-7.3 (3H, m), 8.5-8.6 (1H, m).

Example 19

N-(2-Furylmethyl)-3-{bis[4-(methoxymethoxy)phenyl]methyl}-1-(2-dimethylaminoethyl)indole-2-carboxamide (Compound 19)

$^1$H-NMR($CDCl_3$) δ(ppm): 2.08 (6H, s), 2.76 (2H, t, J=6.3Hz), 3.46 (6H, s), 4.40 (2H, t, J=6.3Hz), 4.56 (2H, d, J=4.7Hz), 5.13 (4H, s), 6.07 (1H, s), 6.23 (1H, d, J=2.0Hz), 6.3-6.35 (1H, m), 6.85-6.95 (1H, m), 6.89 (4H, d, J=8.7Hz), 7.0-7.1 (1H, m), 7.08 (4H, d, J=8.7Hz), 7.15-7.35 (2H, m), 7.37 (1H, br s), 8.4-8.45 (1H, m).

Example 20

N-[2-(1-Methyl-2-pyrrolyl)ethyl]-3-{bis[4-(methoxymethoxy)phenyl]methyl}-1-(2-dimethylaminoethyl)indole-2-carboxamide (Compound 20)

$^1$H-NMR($CDCl_3$) δ(ppm): 2.09 (6H, s), 2.73 (2H, t, J=6.3Hz), 2.80 (2H, t, J=6.7Hz), 3.46 (6H, s), 3.47 (3H, s), 3.60 (2H, q, J=6.7Hz), 4.37 (2H, t, J=6.3Hz), 5.14 (4H, s), 5.75-5.8 (1H, m), 5.95-6.0 (1H, m), 6.07 (1H, s), 6.45-6.5 (1H, m), 6.85-7.05 (2H, m), 6.91 (4H, d, J=8.6Hz), 7.08 (4H, d, J=8.6Hz), 7.15-7.25 (1H, m), 7.29 (1H, d, J=8.3Hz), 7.85-7.95 (1H, m).

Example 21

N-(2-Dimethylaminoethyl)-3-{bis(4-hydroxyphenyl)methyl}-1-(2-dimethylaminoethyl)indole-2-carboxamide (Compound 21)

Compound 1 (2.48 g, 4.21 mmol) obtained in Example 1 was dissolved in ethanol (50 ml), and 15 ml of 2N hydrochloric acid was added thereto, followed by heating under reflux for 2 hours. After cooling the reaction solution, a saturated aqueous solution of sodium bicarbonate was added thereto for neutralization. A little amount of ethyl acetate was added to the mixture followed by stirring at room temperature. The resulting crystals were collected by filtration, washed with water, and dried under reduced pressure to give 1.6 g (yield: 81%) of the title compound.

$^1$H-NMR(DMSO-$d_6$) δ(ppm): 2.11 (6H, s), 2.15 (6H, s), 2.36 (2H, t, J=6.4Hz), 2.5-2.6 (2H, m), 3.3-3.4 (2H, m), 4.32 (2H, t, J=6.4Hz), 5.78 (1H, s), 6.62 (4H, d, J=8.6Hz), 6.83 (1H, t, J=7.5Hz), 6.9-7.0 (1H, m), 6.93 (4H, d, J=8.6Hz), 7.12 (1H, t, J=7.5Hz), 7.4-7.45 (1H, m), 8.15-8.25 (1H, m), 9.05-9.15 (2H, br).

In the following Examples 22 to 39, substantially the same procedure as in Example 21 was repeated using corresponding Compounds 2 to 19 in place of Compound 1 to give the desired compounds.

Example 22

N-(2-Dimethylaminoethyl)-N-methyl-3-[bis(4-hydroxyphenyl)methyl]-1-(2-dimethylaminoethyl)indole-2-carboxamide (Compound 22)

$^1$H-NMR(DMSO-$d_6$) δ(ppm): 2.17 (6H, s), 2.22 (6H, s), 2.4-2.55 (4H, m), 2.63 (3H, s), 3.35-3.65 (2H, m), 4.0-4.3 (2H, m), 5.47 (1H, s), 6.58 (2H, d, J=8.6Hz), 6.65 (2H, d, J=8.6Hz), 6.8-6.9 (3H, m), 7.0-7.15 (4H, m), 7.39 (1H, d, J=8.3Hz), 9.03 (1H, s), 9.10 (1H, s).

Example 23

N-[2-(1-Pyrrolidinyl)ethyl]-3-[bis(4-hydroxyphenyl)methyl]-1-(2-dimethylaminoethyl)indole-2-carboxamide (Compound 23)

$^1$H-NMR(DMSO-$d_6$) δ(ppm): 1.55-1.7 (4H, m), 2.12 (6H, s), 2.35-2.5 (4H, m), 2.5-2.65 (4H, m), 3.3-3.45 (2H, m),

4.25-4.35 (2H, m), 5.82 (1H, s), 6.62 (4H, d, J=8.4Hz), 6.83 (1H, t, J=7.6Hz), 6.94 (4H, d, J=8.4Hz), 7.01 (1H, d, J=8.1Hz), 7.12 (1H, t, J=7.6Hz), 7.39 (1H, d, J=8.6Hz), 8.0-8.1 (1H, br), 9.05 (2H, s).

Example 24

N-[3-(2-Oxo-1-pyrrolidinyl)propyl]-3-[bis(4-hydroxyphenyl)methyl]-1-(2-dimethylaminoethyl)indole-2-carboxamide (Compound 24)

$^1$H-NMR(DMSO-$d_6$) δ(ppm): 1.6-1.75 (2H, m), 1.85-2.0 (2H, m), 2.12 (6H, s), 2.23 (2H, t, J=7.9Hz), 2.5-2.6 (2H, m), 3.15-3.25 (4H, m), 3.25-3.35 (2H, m), 4.29 (2H, t, J=6.3Hz), 5.77 (1H, s), 6.62 (4H, d, J=8.4Hz), 6.8-6.85 (1H, m), 6.93 (4H, d, J=8.4Hz), 6.9-7.0 (1H, m), 7.1-7.15 (1H, m), 7.42 (1H, d, J=8.3Hz), 8.4-8.5 (1H, m), 9.13 (2H, s).

Example 25

N-(2-Piperidinoethyl)-3-[bis(4-hydroxyphenyl)methyl]-1-(2-dimethylaminoethyl)indole-2-carboxamide (Compound 25)

$^1$H-NMR(DMSO-$d_6$) δ(ppm): 1.3-1.5 (6H, m), 2.11 (6H, s), 2.25-2.35 (4H, m), 2.40 (2H, t, J=6.3Hz), 2.57 (2H, t, J=6.4Hz), 3.3-3.4 (2H, m), 4.34 (2H, t, J=6.4Hz), 5.81 (1H, s), 6.62 (4H, d, J=8.2Hz), 6.8-6.9 (1H, m), 6.93 (4H, d, J=8.2Hz), 6.99 (1H, d, J=7.9Hz), 7.1-7.2 (1H, m), 7.42 (1H, d, J=8.3Hz), 8.1-8.2 (1H, m), 9.10 (2H, s).

Example 26

N-(2-Morpholinoethyl)-3-[bis(4-hydroxyphenyl)methyl]-1-(2-dimethylaminoethyl)indole-2-carboxamide (Compound 26)

$^1$H-NMR(DMSO-$d_6$) δ(ppm): 2.11 (6H, s), 2.3-2.4 (4H, m), 2.44 (2H, t, J=6.3Hz), 2.57 (2H, t, J=6.4Hz), 3.39 (2H, t, J=6.3Hz), 3.45-3.5 (4H, m), 4.34 (2H, t, J=6.4Hz), 5.81 (1H, s), 6.63 (4H, d, J=8.4Hz), 6.83 (1H, t, J=7.6Hz), 6.94 (4H, d, J=8.4Hz), 6.99 (1H, d, J=7.6Hz), 7.12 (1H, t, J=7.6Hz), 7.42 (1H, d, J=7.6Hz), 8.26 (3H, s).

Example 27

N-(3-Morpholinopropyl)-3-[bis(4-hydroxyphenyl)methyl]-1-(2-dimethylaminoethyl)indole-2-carboxamide (Compound 27)

$^1$H-NMR(DMSO-$d_6$) δ(ppm): 1.55-1.7 (2H, m), 2.13 (6H, s), 2.25-2.35 (6H, m), 2.5-2.6 (2H, m), 3.2-3.35 (2H, m), 3.5-3.6 (4H, m), 4.28 (2H, t, J=6.7Hz), 5.80 (1H, s), 6.62 (4H, d, J=8.4Hz), 6.8-6.9 (1H, m), 6.9-7.0 (1H, m), 6.93 (4H, d, J=8.4Hz), 7.05-7.15 (1H, m), 7.42 (1H, d, J=8.4Hz), 8.37 (1H, t,J=5.4Hz), 9.12 (2H, s).

Example 28

N-(2-Pyridylmethyl)-3-[bis(4-hydroxyphenyl)methyl]-1-(2-dimethylaminoethyl)indole-2-carboxamide (Compound 28)

$^1$H-NMR(DMSO-$d_6$) δ(ppm): 2.07 (6H, s), 2.62 (2H, t, J=6.3Hz), 4.38 (2H, t, J=6.3Hz), 4.59 (2H, d, J=5.6Hz), 5.93 (1H, s), 6.62 (4H, d, J=8.5Hz), 6.84 (1H, t, J=7.4Hz), 6.9-7.0 (1H, m), 6.92 (4H, d, J=8.5Hz), 7.13 (1H, t, J=7.6Hz), 7.2-7.3 (2H, m), 7.39 (1H, d, J=8.6Hz), 7.69 (1H, td, J=7.6, 1.6Hz), 8.49 (1H, d, J=5.0Hz), 9.03 (2H, s), 9.15 (1H, t, J=5.6Hz).

Example 29

N-(3-Pyridylmethyl)-3-[bis(4-hydroxyphenyl)methyl]-1-(2-dimethylaminoethyl)indole-2-carboxamide (Compound 29)

$^1$H-NMR(DMSO-$d_6$) δ(ppm): 2.03 (6H, s), 2.45-2.55 (2H, m), 4.2-4.3 (2H, m),4.47 (2H, d, J=5.6Hz), 5.77 (1H, s), 6.61 (4H, d, J=8.2Hz), 6.8-6.95 (2H, m), 6.87 (4H, d, J=8.2Hz), 7.05-7.15 (1H, m), 7.25-7.35 (1H, m), 7.42 (1H, d, J=8.3Hz), 7.67 (1H, d, J=8.4Hz), 8.47 (1H, d, J=4.6Hz), 8.57 (1H, s), 9.1-9.2 (1H, m), 9.13 (2H, s).

Example 30

N-(4-Pyridylmethyl)-3-[bis(4-hydroxyphenyl)methyl]-1-(2-dimethylaminoethyl)indole-2-carboxamide (Compound 30)

$^1$H-NMR(DMSO-$d_6$) δ(ppm): 2.06 (6H, s), 2.5-2.6 (2H, m), 4.31 (2H, t, J=6.4Hz), 4.48 (2H, d, J=5.6Hz), 5.87 (1H,

s), 6.63 (4H, d, J=8.6Hz), 6.8-6.95 (2H, m), 6.90 (4H, d, J=8.6Hz), 7.1-7.25 (3H, m), 7.35-7.45 (1H, m), 8.45-8.5 (2H, m), 9.0-9.15 (2H, br), 9.25-9.35 (1H, m).

Example 31

N-[2-(2-Pyridyl)ethyl]-3-[bis(4-hydroxyphenyl)methyl]-1-(2-dimethylaminoethyl)indole-2-carboxamide (Compound 31)

$^1$H-NMR(DMSO-$d_6$) δ(ppm): 2.02 (6H, s), 2.45-2.55 (2H, m), 2.98 (2H, t, J=6.9Hz), 3.65 (2H, q, J=6.4Hz), 4.25 (2H, t, J=6.4Hz), 5.77 (1H, s), 6.62 (4H, d, J=8.4Hz), 6.75-7.0 (2H, m), 6.89 (4H, d, J=8.4Hz), 7.05-7.2 (3H, m), 7.39 (1H, d, J=8.4Hz), 7.59 (1H, td, J=7.6, 2.0Hz), 8.43 (1H, d, J=4.9Hz), 8.59 (1H, t, J=5.4Hz), 9.09 (2H, s).

Example 32

N-[2-(4-Pyridyl)ethyl]-3-[bis(4-hydroxyphenyl)methyl]-1-(2-dimethylaminoethyl)indole-2-carboxamide (Compound 32)

$^1$H-NMR(DMSO-$d_6$) δ(ppm): 1.96 (6H, s), 2.45-2.55 (2H, m), 2.83 (2H, t, J=6.6Hz), 3.55 (2H, q, J=6.6Hz), 4.23 (2H, t, J=6.2Hz), 5.80 (1H, s), 6.63 (4H, d, J=8.3Hz), 6.8-7.0 (2H, m), 6.89 (4H, d, J=8.3Hz), 7.05-7.15 (3H, m), 7.39 (1H, d, J=8.3Hz), 8.36 (2H, d, J=5.9Hz), 8.7-8.8 (1H, m), 9.11 (2H, s).

Example 33

N-[(1-Ethyl-2-pyrrolidinyl)methyl]-3-[bis(4-hydroxyphenyl)methyl]-1-(2-dimethylaminoethyl)indole-2-carboxamide (Compound 33)

$^1$H-NMR(DMSO-$d_6$) δ(ppm): 0.98 (3H, t, J=7.1Hz), 1.45-1.85 (4H, m), 2.0-2.2 (2H, m), 2.12 (6H, s), 2.5-2.6 (3H, m), 2.75-2.85 (1H, m), 2.95-3.05 (1H, m), 3.05-3.2 (1H, m), 3.4-3.5 (1H, m), 4.31 (2H, t, J=6.3Hz), 5.78 (1H, s),6.62 (4H, d, J=8.3Hz), 6.83 (1H, t, J=7.2Hz), 6.9-7.0 (1H, m), 6.93 (4H, d, J=8.3Hz), 7.12 (1H, t, J=7.3Hz), 7.42 (1H, d, J=7.3Hz), 8.0-8.1 (1H, m), 9.13 (2H, s).

Example 34

N-[2-(1-Methyl-2-pyrrolidinyl)ethyl]-3-[bis(4-hydroxyphenyl)methyl]-1-(2-dimethylaminoethyl)indole-2-carboxamide (Compound 34)

$^1$H-NMR(DMSO-$d_6$) δ(ppm): 1.35-1.5 (2H, m), 1.5-1.7 (2H, m), 1.75-1.9 (2H, m), 1.95-2.05 (2H, m), 2.14 (6H, s), 2.17 (3H, s), 2.58 (2H, t, J=6.6Hz), 2.85-2.95 (1H, m), 3.25-3.35 (2H, m), 4.30 (2H, t, J=6.6Hz), 5.77 (1H, s), 6.62 (4H, d, J=8.4Hz), 6.75-6.85 (1H, m), 6.9-7.0 (1H, m), 6.93 (4H, d, J=8.4Hz), 7.11 (1H, t, J=7.3Hz), 7.40 (1H, d, J=8.2Hz), 8.3-8.4 (1H, m), 9.09 (2H, s).

Example 35

N-[3-(2-Methylpiperidino)propyl]-3-[bis(4-hydroxyphenyl)methyl]-1-(2-dimethylaminoethyl)indole-2-carboxamide (Compound 35)

$^1$H-NMR(DMSO-$d_6$) δ(ppm): 0.94 (3H, d, J=6.3Hz), 1.05-1.3 (2H, m), 1.3-1.7 (6H, m), 1.95-2.1 (1H, m), 2.13 (6H, s), 2.1-2.3 (2H, m), 2.5-2.6 (2H, m), 2.6-2.75 (2H, m), 3.2-3.3 (2H, m), 4.28 (2H, t, J=6.6Hz), 5.74 (1H, s), 6.62 (4H, d, J=8.6Hz), 6.8-6.9 (1H, m), 6.9-7.0 (1H, m), 6.92 (4H, d, J=8.6Hz), 7.05-7.15 (1H, m), 7.43 (1H, d, J=8.3Hz), 8.3-8.4 (1H, br), 9.14 (2H, brs).

Example 36

N-[3-(1-Imidazolyl)propyl]-3-[bis(4-hydroxyphenyl)methyl]-1-(2-dimethylaminoethyl)indole-2-carboxamide (Compound 36)

$^1$H-NMR($CDCl_3$-$CD_3OD$) δ(ppm): 1.9-2.0 (2H, m), 2.13 (6H, s), 2.58 (2H, t, J=6.7Hz), 3.21 (2H, t, J=6.9Hz), 3.98 (2H, t, J=6.9Hz), 4.29 (2H, t, J=6.7Hz), 5.78 (1H, s), 6.64 (4H, d, J=8.4Hz), 6.8-6.9 (1H, m), 6.9-7.0 (2H, m), 6.95 (4H, d, J=8.4Hz), 7.1-7.2 (1H, m), 7.16 (1H, s), 7.43 (1H, d, J=8.4Hz), 7.64 (1H, s).

Example 37

N-(2-Tetrahydrofuranylmethyl)-3-[bis(4-hydroxyphenyl)methyl]-1-(2-dimethylaminoethyl)indole-2-carboxamide (Compound 37)

$^1$H-NMR(DMSO-$d_6$) δ(ppm): 1.5-1.6 (1H, m), 1.75-2.0 (3H, m), 2.12 (6H, s), 2.6-2.7 (2H, m), 3.3-3.4 (2H, m), 3.6-3.8 (2H, m), 3.9-4.0 (1H, m), 4.33 (2H, t, J=6.3Hz), 5.86 (1H, s), 6.62 (4H, d, J=8.3Hz), 6.83 (1H, t, J=7.9Hz),6.9-7.0 (5H, m), 7.13 (1H, t, J=7.9Hz), 7.37 (1H, d, J=7.9Hz), 8.66 (1H,t, J=5.6Hz), 9.02 (2H, s).

Example 38

N-(2-Thienylmethyl)-3-[bis(4-hydroxyphenyl)methyl]-1-(2-dimethylaminoethyl)indole-2-carboxamide (Compound 38)

$^1$H-NMR(DMSO-$d_6$) δ(ppm): 2.05 (6H, s), 2.45-2.55 (2H, m), 4.27 (2H, t, J=6.6Hz), 4.62 (2H, d, J=5.6Hz), 5.78 (1H, s), 6.61 (4H, d, J=8.2Hz), 6.8-6.9 (1H, m), 6.89 (4H, d, J=8.2Hz), 6.95-7.05 (3H, m), 7.12 (1H, t, J=7.6Hz), 7.35-7.45 (2H, m), 9.14 (2H, s), 9.29 (1H, t, J=5.6Hz).

Example 39

N-(2-Furylmethyl)-3-[bis(4-hydroxyphenyl)methyl]-1-(2-dimethylaminoethyl)indole-2-carboxamide (Compound 39)

$^1$H-NMR(DMSO-$d_6$) δ(ppm): 2.05 (6H, s), 2.45-2.55 (2H, m), 4.28 (2H, t, J=6.3Hz), 4.46 (2H, d, J=5.6Hz), 5.77 (1H, s), 6.28 (1H, d, J=3.0Hz), 6.41 (1H, br s), 6.61 (4H, d, J=8.6Hz), 6.8-7.0 (2H, m), 6.89 (4H, d, J=8.6Hz), 7.1-7.15 (1H, m), 7.43 (1H, d, J=8.3Hz), 7.59 (1H, br s), 9.1-9.2 (1H, m), 9.14 (2H, s).

Reference Example 1

Ethyl 3-{bis[4-(methoxymethoxy)phenyl]methyl}indole-2-carboxylate

A solution of boron trifluoride-ether complex (0.4 ml, 3.3 mmol) in 10 ml of methylene chloride was added dropwise at room temperature to a solution of 4,4'-bis(methoxymethoxy)benzhydrol (6.22 g, 32.9 mmol) and ethyl indole-2-carboxylate (10.0g, 32.9 mmol) in 100 ml of methylene chloride over 5 minutes, followed by stirring at room temperature for 60 minutes. A saturated aqueous solution of sodium bicarbonate was added to the reaction solution followed by extraction with chloroform. The resulting organic layer was washed with a saturated aqueous solution of sodium chloride and dried over magnesium sulfate, and the solvent was distilled off under reduced pressure to give a crude product. The obtained crude product was recrystallized from ethanol to give 12.9 g (yield: 89%) of the title compound.

$^1$H-NMR($CDCl_3$) δ(ppm): 1.39 (3H, t, J=7.3Hz), 3.46 (6H, s), 4.39 (2H, q, J=7.3Hz), 5.14 (4H, s), 6.58 (1H, s), 6.85-6.95 (5H, m), 7.03 (1H, d, J=7.3Hz), 7.13 (4H, d, J=8.2Hz), 7.2-7.25 (1H, m), 7.36 (1H, d, J=8.6Hz), 8.80 (1H, s).

Reference Example 2

Ethyl 3-{bis[4-(methoxymethoxy)phenyl]methyl}-1-(2-dimethylaminoethyl)indole-2-carboxylate

To a suspension of ethyl 3-{bis[4-(methoxymethoxy)phenyl]methyl}indole-2-carboxylate (12.9 g, 27.0 mmol) obtained in Reference Example 1 in 70 ml of N,N-dimethylformamide was added sodium hydride (60% in oil, 2.27 g, 56.8 mmol), and then 2-dimethylaminoethylchloride hydrochloride (3.98 g, 27.6 mmol) was added thereto, followed by heating to 80°C and then stirring for one hour. A saturated aqueous solution of ammonium chloride was added to the reaction solution for neutralization, and water was added thereto followed by extraction with ethyl acetate. The resulting organic layer was washed successively with an aqueous solution of ammonium chloride, a saturated aqueous solution of sodium bicarbonate, and a saturated aqueous solution of sodium chloride, and dried over magnesium sulfate, and the solvent was distilled off under reduced pressure to give 16.4 g (quantitative) of the title compound as a crude product, which was used as a starting material for Reference Example 3 without further purification.

$^1$H-NMR($CDCl_3$) δ(ppm): 1.37 (3H, t, J=7.2Hz), 2.34 (6H, s), 2.6-2.75 (2H, m), 3.45 (6H, s), 4.35 (2H, q, J=7.2Hz), 4.5-4.65 (2H, m), 5.13 (4H, s), 6.47 (1H, s), 6.8-7.05 (6H, m), 7.09 (4H, d, J=8.5Hz), 7.1-7.2 (1H, m), 7.39 (1H, d, J=8.6Hz).

Reference Example 3

3-{Bis[4-(methoxymethoxy)phenyl]methyl}-1-(2-dimethylaminoethyl)indole-2-carboxylic acid

The crude ethyl 3-{bis[4-(methoxymethoxy)phenyl]methyl}-1-(2-dimethylaminoethyl)indole-2-carboxylate (16.4 g, approximately 27.0 mmol) obtained in Reference Example 2 was dissolved in 100 ml of ethanol, and a 2N aqueous sodium hydroxide solution (40 ml) was added thereto, followed by heating under reflux for 3 hours. The solvent was distilled off under reduced pressure, water was added to dissolve the residue, and the pH of the solution was adjusted to 5 by adding 2N hydrochloric acid, followed by extraction with chloroform. The resulting organic layer was washed with water and a saturated aqueous solution of sodium chloride, and dried over magnesium sulfate, and the solvent was distilled off under reduced pressure to give 14.4 g of a crude product. The obtained crude product was crystallized from ethyl acetate to give 8.9 g (yield: 64%) of the title compound.

$^1$H-NMR($CDCl_3$) δ(ppm): 2.39 (6H, s), 3.18 (2H, t, J=6.0Hz), 3.45 (6H, s), 4.74 (2H, t, J=6.0Hz), 5.12 (4H, s), 6.68 (1H, s), 6.88 (4H, d, J=8.9Hz), 6.9-7.0 (1H, m), 7.15-7.25 (3H, m), 7.18 (4H, d, J=8.9Hz).

Industrial Applicability

According to the present invention, there can be provided indole derivatives which are useful as therapeutic agents for osteoporosis.

**Claims**

1. An indole derivative represented by formula (I):

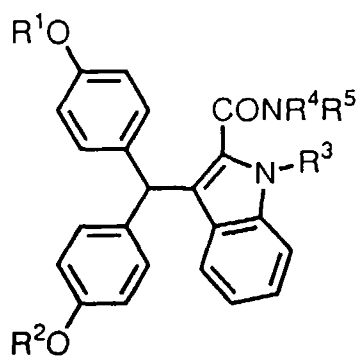

(I)

wherein $R^1$ and $R^2$ independently represent hydrogen, lower alkyl, $-O-(CH_2)_n-OR^6$ (wherein $R^6$ represents hydrogen or lower alkyl, and n represents an integer of 1 to 6), or

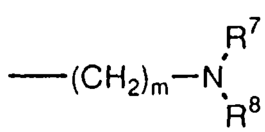

(wherein $R^7$ and $R^8$ independently represent hydrogen or lower alkyl, or $R^7$ and $R^8$ are combined together with the adjacent nitrogen atom to form a substituted or unsubstituted alicyclic heterocyclic group, and m represents an integer of 2 to 6), $R^3$ represents hydrogen, lower alkyl, or

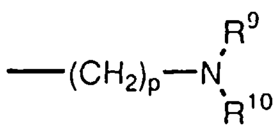

(wherein $R^9$ and $R^{10}$ independently represent hydrogen or lower alkyl, or $R^9$ and $R^{10}$ are combined together with the adjacent nitrogen atom to form a substituted or unsubstituted alicyclic heterocyclic group, and p represents an integer of 2 to 6), $R^4$ represents hydrogen, lower alkyl, alicyclic alkyl, substituted or unsubstituted aryl, a substituted

or unsubstituted heterocyclic group,

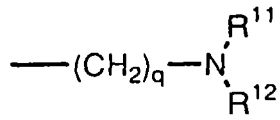

(wherein $R^{11}$ and $R^{12}$ independently represent hydrogen or lower alkyl, or $R^{11}$ and $R^{12}$ are combined together with the adjacent nitrogen atom to form a substituted or unsubstituted alicyclic heterocyclic group, and q represents an integer of 2 to 6), or $-(CH_2)_r-R^{13}$ (wherein $R^{13}$ represents substituted or unsubstituted aryl or a substituted or unsubstituted heterocyclic group, and r represents an integer of 1 to 6), and $R^5$ represents

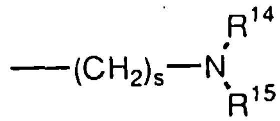

(wherein $R^{14}$ and $R^{15}$ independently represent hydrogen or lower alkyl, or $R^{14}$ and $R^{15}$ are combined together with the adjacent nitrogen atom to form a substituted or unsubstituted alicyclic heterocyclic group, and s represents an integer of 2 to 6), or $-(CH_2)_t-R^{16}$ (wherein $R^{16}$ represents substituted or unsubstituted aryl or a substituted or unsubstituted heterocyclic group, and t represents an integer of 1 to 6), or a pharmaceutically acceptable salt thereof.

**2.** An indole derivative according to claim 1, wherein $R^1$ and $R^2$ represent hydrogen, or a pharmaceutically acceptable salt thereof.

**3.** An indole derivative according to claim 2, wherein $R^3$ represents

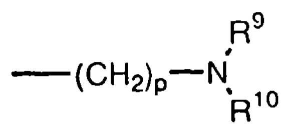

(wherein $R^9$, $R^{10}$, and p have the same meanings as defined above), or a pharmaceutically acceptable salt thereof.

**4.** A pharmaceutically acceptable composition comprising a pharmaceutically acceptable carrier and an effective amount of a compound according to any one of claims 1 to 3 as an active ingredient.

## INTERNATIONAL SEARCH REPORT

International application No.
PCT/JP96/01979

**A. CLASSIFICATION OF SUBJECT MATTER**

Int. $Cl^6$ C07D209/42, 401/06, 403/06, 405/06, 409/06, A61K31/40, 31/41, 31/44, 31/445, 31/535

According to International Patent Classification (IPC) or to both national classification and IPC

**B. FIELDS SEARCHED**

Minimum documentation searched (classification system followed by classification symbols)

Int. $Cl^6$ C07D209/42, 401/06, 403/06, 405/06, 409/06, A61K31/40, 31/41, 31/44, 31/445, 31/535

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| P,X | WO, 95/19343, A1 (Kyowa Hakko Co., Ltd.), July 20, 1995 (20. 07. 95), Full descriptions & AU, 9514247, A | 1 - 4 |
| A | J. Med. Chem., 17(12), (1974), p. 1298-1304, Calvert W. Whitehead et al., "Effect of lipophilic substituents on some biological properties of indoles" | 1 - 4 |

☐ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| October 2, 1996 (02. 10. 96) | October 15, 1996 (15. 10. 96) |
| Name and mailing address of the ISA/ Japanese Patent Office | Authorized officer |
| Facsimile No. | Telephone No. |

Form PCT/ISA/210 (second sheet) (July 1992)